(19) Europäisches Patentamt
European Patent Office
Office européen des brevets

(11) Veröffentlichungsnummer: 0 147 593
B1

(12) # EUROPÄISCHE PATENTSCHRIFT

(45) Veröffentlichungstag der Patentschrifft: **28.02.90**

(51) Int. Cl.[5]: **C 07 C 45/40**, C 07 C 67/313, C 07 C 41/50

(21) Anmeldenummer: **84113702.9**

(22) Anmeldetag: **14.11.84**

(54) **Verfahren zur Herstellung von Mono- oder Biscarbonylverbindungen.**

(30) Priorität: **23.12.83 AT 4500/83**

(43) Veröffentlichungstag der Anmeldung:
**10.07.85 Patentblatt 85/28**

(45) Bekanntmachung des Hinweises auf die Patenterteilung:
**28.02.90 Patentblatt 90/09**

(84) Bennante Vertragsstaaten:
**AT BE CH DE FR GB IT LI LU NL SE**

(56) Entgegenhaltungen:
**DE-A-3 346 266**
**GB-A-1 382 349**
**US-A-2 733 270**
**US-A-3 705 922**

**Die Akte enthält technische Angaben, die nach dem Eingang der Anmeldung eingereicht wurden und die nicht in dieser Patentschrift enthalten sind.**

(73) Patentinhaber: **Chemie Linz Gesellschaft m.b.H.**
**St.Peter-Strasse 25**
**A-4021 Linz (AT)**

(72) Erfinder: **Sajtos, Alexander, Dipl.-Ing.**
**Hartackerstrasse 48**
**A-4020 Linz (AT)**
Erfinder: **Wechsberg, Manfred, Dipl.-Ing. Dr.**
**Im Blumengrund 9/35**
**A-4020 Linz (AT)**
Erfinder: **Roithner, Erich**
**Stülzgasse 14**
**A-4020 Linz (AT)**
Erfinder: **Pollhammer, Stefan**
**In der Neupeint 20**
**A-4020 Linz (AT)**
Erfinder: **Mahringer, Andreas**
**Hallestrasse 51**
**A-4020 Linz (AT)**

(74) Vertreter: **Kunz, Ekkehard, Dr.**
**Chemie Holding AG Patentwesen St. Peter-Strasse 25**
**A-4021 Linz (AT)**

EP 0 147 593 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

## Beschreibung

Die Erfindung betrifft ein Verfahren zur Herstellung von Mono- oder Biscarbonylverbindungen aus ungesättigten organischen Kohlenstoffverbindungen mit einer oder mehreren olefinischen oder aromatischen Doppelbindungen im Molekül.

Die Herstellung von Carbonylverbindungen aus organischen Verbindungen, die als Strukturelement eine oder mehrere C = C-Doppelbindungen im Molekül aufweisen, mittels eines zweistufigen Ozonolyse- und Reduktionsprozesses ist bekannt. Bei der Durchführung dieser Methode wird in der ersten Stufe zur Erzielung einer möglichst vollständigen Ozonisierung der Doppelbindung meistens mit einem Ozonüberschuß gearbeitet. Die in der zweiten Stufe folgende reduktive Spaltung bereitet immer wieder Schwierigkeiten, da die peroxidhältigen Ozonisierungsprodukte instabil sind und in Anwesenheit von metallischen Hydrierkatalysatoren besonders leicht Umlagerungen erfahren, bevor sie zu den entsprechenden Carbonylverbindungen reduziert werden können. Außerdem werden bei Edelmetallkatalysatoren im längeren Kontakt mit peroxidhältigen Lösungen Aktivitätsverluste des Katalysators beobachtet, sodaß die Lösungen bei der reduktiven Spaltung durch Hydrierung in der Regel nicht gänzlich peroxidfrei werden und neben Schwierigkeiten bei der Reindarstellung der Endprodukte auch Ausbeuteverluste hingenommen werden müssen.

Zur Vermeidung dieser Schwierigkeiten wird in der US-PS-3 145 232 ein Verfahren zur Herstellung von Carbonylverbindungen empfohlen, bei der die reduktive Spaltung nach der Ozonolyse bei Temperaturen unterhalb von -40°C in Anwesenheit eines Trialkylphosphits durchgeführt wird. Neben dem apparativen Aufwand zur Herstellung der extrem tiefen Reaktionstemperaturen erfordert eine solche Reaktionsführung die Verwendung von absolut wasserfreien Lösungsmitteln, da die Trialkylphosphite in wasserhältigen Lösungsmitteln äußerst rasch hydrolysiert werden. Außerdem bereitet die Abtrennung der freien Carbonylverbindungen von den bei der Reduktion entstehenden Phosphatestern erhebliche Schwierigkeiten.

Da nachgewiesen wurde, daß sich tiefe Reaktionstemperaturen nachteilig auf die Aktivität der eingesetzten Reduktionsmittel auswirken und deshalb Ausbeuteverluste entstehen, wird nach einem Verfahren zur Herstellung von aliphatischen, aromatischen und heteroaromatischen Aldehyden, wie es in der US-PS-3 637 721 beschrieben ist, zwar die Ozonolyse der C = C-Doppelbindung bei -50°C durchgeführt, während die Reaktionstemperaturen im Verlaufe der reduktiven Spaltung der Ozonisierungsprodukte mit aromatischen oder aliphatischen Disulfiden bis auf 50°C gesteigert wird. Beim genannten Verfahren gestaltet sich aber die Abtrennung der bei der Reduktion als Begleitprodukte entstehenden Sulfoxide, beispielsweise Dimethylsulfoxid, von den als Verfahrensprodukte entstehenden Aldehyden als äußerst schwierig und ist in vielen Fällen ohne Derivatisierung der Aldehyde überhaupt undurchführbar.

In der US-PS-3 705 922 oder der DE-OS-2 514 001 ist schließlich die Herstellung von Carbonylverbindungen mittels eines Ozonolyse- und Reduktionsverfahrens beschrieben, bei dem die als Ausgangsmaterial dienenden ungesättigten Verbindungen mit einem Überschuß an Ozon umgesetzt und die dabei gebildeten Ozonisierungsprodukte durch katalytische Hydrierung reduktiv gespalten werden. Dabei muß überschüssiges Ozon aber vor der reduktiven Aufspaltung zum Schutz des Hydrierkatalysators vor Aktivitätsverlusten durch Spülung der Reaktionslösung mit einem Inertgas, beispielsweise mit Stickstoff, in einem eigenen Arbeitsgang wieder entfernt werden.

Zur Durchführung der Hydrierung setzt man dann dem bei der Ozonolyse gebildeten Reaktionsgemisch den Katalysator, der bevorzugt ein Edelmetallkatalysator ist, direkt zu und leitet bis zur Sättigung Wasserstoff ein.

Da Edelmetallkatalysatoren bei längerem Kontakt mit organischen Peroxiden desaktiviert werden, hängt bei den bekannten Verfahren die Ausbeute bei der Hydrierung von der Menge des jeweils eingesetzten Hydrierkatalysators ab. Wie aus einem Vergleich der Beispiele in der US-PS-3 705 922 hervorgeht, nimmt die Ausbeute trotz entsprechend verlängerter Reaktionszeit um etwa 10 % ab, wenn bei gleicher Ansatzgröße anstelle von 0.5 g nur 0.2 g eines $Pd/Al_2O_3$ Katalysators verwendet werden. In den genannten Druckschriften finden sich aber auch keine Angaben über die Möglichkeiten zur Regenerierung oder Wiederverwendung der eingesetzten Edelmetallkatalysatoren nach Beendigung der Hydrierung.

Es wurde nun überraschenderweise gefunden, daß die den bekannten Verfahren anhaftenden Nachteile gemäß der vorliegenden Erfindung durch ein einfaches und ökonomisches Verfahren vermieden werden können, bei dem man eine ungesättigte organische Kohlenstoffverbindung mit einer oder mehreren olefinischen oder aromatischen Doppelbindungen unter Vermeidung jeglichen Überschusses mit einem Moläquivalent Ozon umsetzt und anschließend die peroxidhältigen Ozonisierungsprodukte in verdünnter Lösung bei einer niedrigen Konzentration an Peroxiden durch katalytische Hydrierung rasch reduktiv spaltet.

Im Vergleich zu den bekannten Verfahren liefert das Verfahren nach der Erfindung auf einfacherem und wirtschaftlicherem Weg Carbonylverbindungen in besserer Ausbeute und Reinheit. Die Katalysatoren werden beim erfindungsgemäßen Verfahren geschont und nicht in deutlichem Maß während einer längeren Betriebsdauer vergiftet, sodaß sie auch ohne Regenerierung und Aufarbeitung bei der Wiederverwendung keinen merkbaren Aktivitätsverlust zeigen. Alle diese vorteilhaften Eigenschaften sind im Hinblick auf den Stand der Technik überraschend.

Gegenstand der vorliegenden Erfindung ist demnach ein Verfahren zur Herstellung von Mono- oder Biscarbonylverbindungen der allgemeinen Formel

$Q - X - C(=O) - R$ I, I

worin

Q Wasserstoff oder die Reste $-C(=O)-H$, $-C(=O)-R_1$, $-CH(OR_1)_2$, $-C(=O)-OR_1$ bezeichnet, mit der Bedeutung für $R_1$ ist $C_1$ bis $C_6$ -Alkyl,

X einen gegebenenfalls ein- oder mehrfach substituierten, geradkettigen oder verzweigten zweiwertigen aliphatischen Alkylenrest mit 1 bis 20 C-Atomen, wobei dieser Alkylenrest durch eine oder mehrere Gruppen, die unter den Reaktionsbedingungen inert sind, substituiert sein kann, einen geradkettigen oder verzweigten aliphatischen Alkylenrest mit 2 bis 20 C-Atomen, wobei eine der $-CH_2$ -Gruppen der Alkylenketten durch ein Sauerstoffatom, ein Schwefelatom oder eine $-SO_2$ -Gruppe ersetzt ist, einen gegebenenfalls ein- oder mehrfach substituierten Phenylenrest, wobei dieser Phenylenrest durch eine oder mehrere Gruppen, die unter den Reaktionsbedingungen inert sind, substituiert sein kann, einen gegebenenfalls ein- oder mehrfach substituierten zweiwertigen Alkylen-, Alkylarylen- oder Alkylenarylenrest mit 7 bis 12 C-Atomen, wobei obgenannte Reste durch eine oder mehrere Gruppen, die unter den Reaktionsbedingungen inert sind, substituiert sein können, einen zweiwertigen Heterocyclus mit einem oder zwei Heteroatomen im Ring oder eine Einfachbindung zwischen zwei benachbarten C-Atomen bedeutet.

R Wasserstoff, einen $C_1$ bis $C_4$-Alkylrest oder den Rest $-C(=O)-OR_1$ bezeichnet, wobei Glyoxal, Alkylglyoxale und deren Dialkylacetale aus der Formel I ausgenommen sind, durch Ozonisierung von ungesättigten organischen Kohlenstoffverbindungen mit einer oder mehreren olefinischen oder aromatischen Doppelbindungen im Molekül und anschließende katalytische Hydrierung der Ozonisierungsprodukte, welches dadurch gekennzeichnet ist, daß man

a) eine ungesättigte Verbindung der allgemeinen Formel

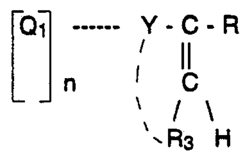

II

worin

n 0 oder 1 ist,

$Q_1$ Wasserstoff oder die Reste

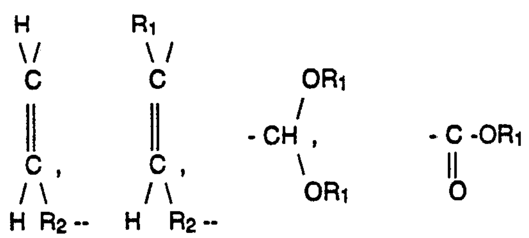

bezeichnet, mit der Bedeutung für $R_1$, ist $C_1$ bis $C_6$-Alkyl,

$R_2$ und $R_3$ unabhängig voneinander für Wasserstoff oder einen $C_1$ bis $C_4$-Alkylrest stehen oder, falls n 1 ist und $Q_1$ den Rest

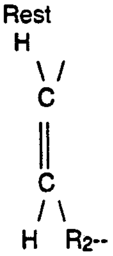

darstellt, stehen $R_2$ und $R_3$ zusammen für eine Einfachbindung zwischen zwei benachbarten C-Atomen oder für einen Alkylenrest mit 2 bis 4 C-Atomen falls Y einen o-Phenylenrest oder einen Alkylenrest mit 2 bis 4 C-Atomen und R ein Wasserstoffatom bedeutet,

Y dieselbe Bedeutung wie X in Formel I hat, falls n 1 bedeutet, oder falls n für 0 steht, zusammen mit $R_3$ einen gegebenenfalls substituierten, zweiwertigen aliphatischen oder araliphatischen Rest, dessen aliphatische Kette durch Sauerstoff oder Schwefel durchbrochen sein kann, bedeutet, und R wie in Formel I definiert ist, in einem niedrigen aliphatischen Alkohol bei Temperaturen von -80°C bis +20°C mit der äquivalenten Menge Ozon umsetzt und

b) die dabei entstehende peroxidhältige Lösung in eine Suspension des Hydrierkatalysators in dem in Stufe a) bei der Ozonisierung verwendeten Alkohol kontinuierlich in einer solchen Dosierung einspeist, das über den ganzen Verlauf der Hydrierung in der Hydrierlösung ein Peroxidgehalt von maximal 0.1 Mol/l eingestellt und/oder aufrechterhalten wird, und die Ozonisierungsprodukte bei pH 2 bis 7 und Temperaturen von 15 bis 45°C unter einem Druck von 1 bis 20 bar durch Hydrierung laufend in die entsprechende Carbonylverbindung reduktiv aufspaltet.

Das erfindungsgemäße Verfahren kann für den Fall der Verwendung von a) Methylmethacrylat oder b) Sulfolen als Ausgangsmaterial durch die folgenden Formeln beschrieben werden:

a) 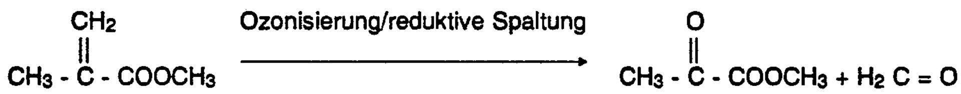


b) 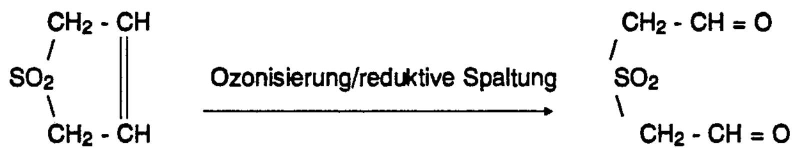


Als Ausgangsprodukte können solche Verbindungen der Formel II zu den dementsprechenden Mono- oder Biscarbonylverbindungen der Formel I umgesetzt werden, bei denen in Y unter einem zweiwertigen aliphatischen Rest beispielsweise ein geradkettiger oder verzweigter Alkylenrest mit 1 bis 20, bevorzugt 2 bis 10 Kohlenstoffatomen, wobei ein $-CH_2-$ Rest in der aliphatischen Kette durch Sauerstoff, Schwefel, beispielsweise durch den $-SO_2-$ Rest ersetzt sein kann, zu verstehen ist, als araliphatischer Rest ein Aralkylen-, Alkylarylen- oder Alkylenarylenrest mit beispielsweise 7 - 12, bevorzugt 7 - 10 Kohlenstoffatomen, als aromatischer Rest beispielsweise ein Phenylenrest und als heteroaromatischer Rest ein zweiwertiger Rest eines beispielsweise fünf-oder sechsgliedrigen Heterocyclus mit einem oder zwei Heteroatomen im Ring genannt sei. Die vorgenannten Reste können noch durch eine oder mehrere unter den Reaktionsbedingungen inerte Gruppen, beispielsweise durch Alkyl-, Alkoxy- oder Alkoxycarbonylgruppen mit jeweils 1 bis 4 Kohlenstoffatomen oder Nitrogruppen substituiert sein.

In bevorzugter Weise werden ungesättigte Verbindungen der Formel

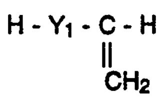 IIa

in der

$Y_1$ zusammen mit Wasserstoff einen in ortho-, meta- oder para-Stellung substituierten Phenylrest oder einen sechsgliedrigen Heteroarylrest mit einem Heteroatom im Ring, besonders bevorzugt aber den para-Nitrophenyl-, p-Tolyl, -2- oder 4-Pyridinylrest darstellt, zu den dementsprechend bevorzugten Carbonylverbindungen umgesetzt. Beispiele für ungesättigte Verbindungen der Formel IIa sind para-Nitro- oder para-Methylstyrol sowie 2- oder 4-Vinylpyridin.

Bevorzugt werden auch ungesättigte Verbindungen der Formel

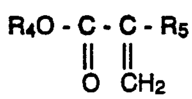 IIb

in der

$R_4$ Methyl oder Äthyl und $R_5$ Methyl, Äthyl oder den Äthoxycarbonylrest bezeichnet, zu den dementsprechend bevorzugten Carbonylverbindungen umgesetzt. Ganz besonders bevorzugt umgesetzt werden Verbindungen in denen $R_4$ und $R_5$ Methyl bedeutet. Beispiele für Ausgangsverbindungen der Formel IIb sind Methylmethacrylat, Alkylacrylsäureäthylester oder Diäthylmethylenmalonat.

Eine weitere bevorzugte Gruppe an Ausgangsprodukten zur Herstellung der dementsprechend bevorzugten Carbonylverbindungen der Formel I sind Verbindungen der Formel

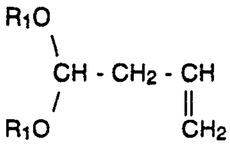

IIc

in der $R_1$, wie in Formel I definiert ist. Beispiele für Verbindungen der Formel IIc sind 4,4-Dimethoxybuten oder 4,4-Di-n-butoxy-buten.

Weiters werden in bevorzugter Weise Verbindungen der Formel

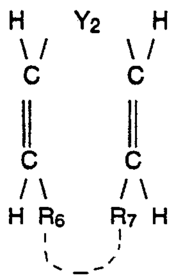

IId

in der $Y_2$ einen o-Phenylenrest oder einen Alkylenrest mit 2 bis 4 C-Atomen und $R_6$ und $R_7$ gemeinsam eine Einfachbindung zwischen den benachbarten C-Atomen oder einen Alkylenrest mit 2 bis 4 C-Atomen bezeichnet, zu den dementsprechend bevorzugten Dialdehyden der Formel I umgesetzt. Beispiele für Verbindungen der Formel IId sind Naphthalin oder Cyclooctadien (1.5).

Schließlich wird in bevorzugter Weise eine weitere Gruppe von ungesättigten Verbindungen der Formel

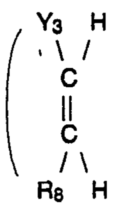

IIe

in der $Y_3$ und $R_8$ zusammen einen Alkylenrest mit 2 bis 6 C-Atomen oder die Reste

$-CH_2-SO_2-CH_2-$, $-CH_2-O-CH_2$, 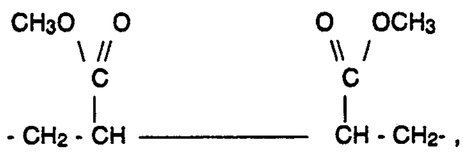 ,

oder 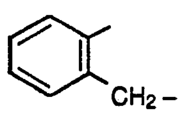 bedeuten, zu den dementsprechend bevorzugten Dialdehyden der Formel I umgesetzt.

Beispiele für Verbindungen der Formel IIe sind Cyclohexen, Cycloocten, Sulfolen, Inden, Tetrahydrophthalsäuredimethylester oder 2,5-Dihydrofuran.

Die Ozonisierung wird vorzugsweise bei Temperaturen von -30 bis 0°C durchgeführt, wobei die Einhaltung einer Temperatur von -15 bis -0°C wiederum besonders bevorzugt ist.

Beim erfindungsgemäßen Verfahren wird die jeweils zur Umsetzung gelangende ungesättigte Verbindung der Formel II mit genau der äquivalenten Menge an Ozon behandelt, wobei unter den angegebenen Verfahrensbedingungen Ozon quantitativ umgesetzt wird und stöchiometrische Mengen des Ausgangsmaterials der Formel II verbraucht werden. Durch die Vermeidung eines Ozonüberschusses kann die während der Ozonisierung von olefinischen Doppelbindungen in einigen Fällen beobachtete Neigung des Reaktionsgemisches zum explosionsartigen Spontanzerfall verhindert werden und es muß nach Beendigung der Ozonisierung auch nicht mehr dafür Sorge getragen werden, daß überschüssiges oder nicht umgesetztes Ozon vor der Hydrierung aus dem Reaktionsgemisch vertrieben wird.

Die Umsetzung der ungesättigten Verbindungen mit Ozon in Stufe a) erfolgt in einem niedrigen aliphatischen Alkohol, in dem die Ausgangsverbindungen gut löslich sind. Bevorzugte Lösungsmittel sind vor allem Methanol oder Äthanol, wobei wiederum die Verwendung von Methanol besonders bevorzugt ist.

Die an die Ozonisierung anschließende katalytische Hydrierung der Ozonolyseprodukte wird beim erfindungsgemäßen Verfahren in stark verdünnter Lösung durchgeführt, wobei durch geeignete Maßnahmen und Vorrichtungen dafür Sorge getragen wird, daß während der gesamten Hydrierung in der Hydrierlösung ein Peroxidgehalt von höchstens 0.1 Mol/l, vorzugsweise von höchstens 0.05 Mol/l und insbesondere von höchstens 0.02 Mol/l eingestellt und aufrecht erhalten wird.

Zur praktischen Durchführung wird beispielsweise in einem Hydrierreaktor eine Suspension des Katalysators in dem in Stufe a) bei der Ozonisierung verwendeten Alkohol, bevorzugt in Methanol oder Äthanol, ganz bevorzugt in Methanol vorgelegt und die bei der Ozonisierung erhaltene Lösung mittels einer regelbaren Dosiervorrichtung kontinuierlich eingespeist. Bei der Zugabe der Ozonolyselösung zu Beginn und im Verlaufe der Hydrierung ist selbstverständlich darauf zu achten, daß durch die zugeführte Menge der peroxidhältigen Ozonisierungsprodukte der oben angegebene Peroxidgehalt in der Hydrierlösung nicht überschritten wird.

Durch die geringe Konzentration an peroxidhältigen Ozonisierungsprodukten während des eigentlichen Hydriervorganges ist das Mengenverhältnis von Katalysator zum zu reduzierendem Substrat über die gesamte Dauer der Hydrierung hinweg gleichmäßig günstig, sodaß auch bei sparsamen Einsatz des Katalysators eine rasche Reduktion gewährleistet ist. Auf diese Weise wird auch die bei hohen Peroxidkonzentrationen sonst zu beobachtende Vergiftung und der damit verbundene Aktivitätsverlust des Katalysators verhindert.

Im gesamten gesehen kann aber durch die kontinuierliche Einspeisung eine große Menge an Ozonisierungsprodukten in einem verhältnismäßig kleinen Volumen reduktiv aufgespalten werden, wodurch in der Endstufe des Verfahrens konzentrierte Lösungen anfallen und neben Lösungsmittel selbst auch Zeit und Kosten bei der destillativen Entfernung der Lösungsmittel während der Aufarbeitung gespart werden.

Als Katalysatoren eignen sich die für Hydrierungen üblicherweise verwendeten Edelmetallkatalysatoren, die in Form von Pulverkontakten mit Trägermaterialien oder ohne Trägermaterial eingesetzt werden können. Bevorzugt werden Palladium- oder Platinkatalysatoren verwendet, insbesondere Platinkatalysatoren ohne Trägermaterial. Bei Pulverkontakten eignen sich als Trägermaterial beispielsweise Kohle, Aluminium, Silikagel oder Kieselgur. Die Ausbeuten sind beim erfindungsgemäßen Verfahren an sich unabhängig von der eingesetzten Katalysatormenge, jedoch empfiehlt sich zur Erzielung einer ausreichenden Hydriergeschwindigkeit die genannten Katalysatoren in Edelmetallmengen von 0.1 bis 5 Gew.-%, vorzugsweise von 0.5 bis 2 Gew.-%, bezogen auf die jeweils pro-Stunde eingespeiste Gesamtmenge an Ozonisierungsprodukten, vorzulegen.

Die Hydrierung wird so lange fortgesetzt, bis keine Wasserstoffaufnahme mehr feststellbar ist. Beim erfindungsgemäßen Verfahren werden zur Reduktion der Ozonisierungsprodukte äquivalente Mengen an Wasserstoff verbraucht. Die Menge an Wasserstoff, die bei der Hydrierung verwendet werden kann, reicht von einem Moläquivalent bis zu einem mehrfachen molaren Überschuß. Die Verwendung von überschüssigem Wasserstoff bringt an sich keine Vorteile und ist nur zweckmäßig, um eine ausreichende Versorgung des Hydriergemisches mit Wasserstoff sicherzustellen.

Die Hydrierung erfolgt beim erfindungsgemäßen Verfahren vorteilhafterweise unter praktisch drucklosen Bedingungen.

Unter praktisch drucklosen Bedingungen sollen hier Drucke von 1 bis etwa 3 bar verstanden werden, wie das in der Technik üblich ist, um das Eindringen von Luft in den Hydrierreaktor zu verhindern. Auf diese Weise ist die Reduktion der Ozonisierungsprodukte in technischer und apparativer Hinsicht sehr einfach durchzuführen. Es ist aber auch möglich, die Hydrierung bei einem Druck bis zu 20 bar durchzuführen und dadurch die Hydrierungsgeschwindigkeit zu steigern.

Die reduktive Spaltung verläuft exotherm und wird gemäß einer bevorzugten Ausführungsform der vorliegenden Erfindung bei 20 bis 40°C, bevorzugt bei Temperaturen im Bereich von 35 bis 40°C durchgeführt.

Vorteilhafterweise wird während der Hydrierung ein pH-Wert von 3 bis 5 eingehalten. Da sich im Verlaufe der Hydrierung in geringen Mengen saure Nebenprodukte bilden, ist zur Einhaltung des gewünschten pH-Wertes die dosierte Zugabe einer Base, vorteilhafterweise von verdünnter Natronlauge, notwendig.

Nach Beendigung der Hydrierung erhält man unter den Bedingungen des erfindungsgemäßen Verfahrens eine alkoholische Lösung der Verfahrensprodukte die gänzlich peroxidfrei ist und in gefahrloser Weise aufgearbeitet werden kann. Vor der Aufarbeitung des Reaktionsgemisches wird der Katalysator nach einer der bekannten Methoden, beispielsweise durch Filtrieren, Abdekantieren oder Zentrifugieren abgetrennt und das Lösungsmittel bevorzugt durch Abdestillieren zurückgewonnen.

Der aus dem Reaktionsgemisch abgetrennte Katalysator wird ohne Regenerierung oder Aufarbeitung für die reduktive Aufspaltung in weiteren Reaktionszyklen verwendet, wobei kein Aktivitätsverlust des Katalysators beobachtet wird. Zweckmäßigerweise kann man dabei so vorgehen, daß der Inhalt des Hydrierreaktors nach beendeter Hydrierung soweit abgesaugt wird, daß eine Restmenge der Hydrierlösung, die beispielsweise ein Fünftel bis ein Zehntel des ursprünglichen Reaktorinhalts beträgt, zusammen mit dem Katalysator im Reaktor zurückbleibt. In diesen Rückstand kann dann eine neue Charge an Ozonisierungsprodukten unter den oben angegebenen Bedingungen eindosiert und unter Wasserstoffzufuhr reduktiv gespalten werden. Überraschenderweise kann die reduktive Spaltung unter den Bedingungen des erfindungsgemäßen Verfahrens in einer großen Anzahl von aufeinanderfolgenden Reaktionszyklen, beispielsweise von 10 bis 100, mit demselben Katalysator bei etwa gleicher Ausbeute und etwa gleichem Wasserstoffverbrauch wie im ersten Reaktionszyklus durchgeführt werden.

In manchen Fällen kann es vorteilhaft sein, vor dem Entfernen der Lösungsmittels die durch die Basenzugabe im Reaktionsgemisch enthaltenen Kationen zu entfernen, beispielsweise durch Behandeln der Lösung mit einem sauren Ionenaustauscher.

Die Aufarbeitung und Reindarstellung der Verfahrensprodukte kann nach den üblichen chemischen Methoden,

beispielsweise durch Rektifizieren, Extrahieren oder Auskristallisieren durchgeführt werden.

Die als Ausgangssubstanzen benötigten ungesättigten Kohlenstoffverbindungen der Formel II sind entweder handelsübliche Substanzen oder in einfacher Weise nach bekannten chemischen Methoden zugänglich. So sind beispielsweise die 2-oder 4-Vinylpyridine der Formel IIa durch Umsetzung von Picolinen mit Formaldehyd leicht erhältlich. Alkylacrylsäurealkylester der Formel IIb, insbesondere Methylmethacrylat als Ausgangsmaterial für die erfindungsgemäße Herstellung von Brenztraubensäuremethylester, sind großtechnisch herstellbare Produkte. Dialkoxybutene der Formel IIc können beispielsweise durch Dimerisierung von Alkylvinyläthern unter Verwendung von katalytischen Mengen $HgF_2$ in sehr guten Ausbeuten in wirtschaftlicher Weise erhalten werden. Die Ausgangsverbindungen der Formeln IId und IIe, beispielsweise Cyclooctadien, Naphthalin, Cycloocten, Cyclohexen, Sulfolen, Inden, Tetrahydrophthalsäuredimethylester oder 2,5-Dihydrofuran sind leicht zugängliche, handelsübliche Substanzen.

Die Verfahrensprodukte der Formel I sind wertvolle Ausgangs- und Zwischenprodukte, aus denen man eine große Anzahl von chemischen Verbindungen unterschiedlicher Struktur oder Substanzen mit beispielsweise großer biologischer und pharmakologischer Bedeutung herstellen kann.

Das erfindungsgemäße Verfahren wird in den folgenden Beispielen näher erläutert.

**Beispiel 1: p-Tolualdehyd**

177 g p-Methylstyrol, gelöst in 1 l Methanol, werden bei -10°C durch Einleiten eines $O_2/O_3$-Gemisches, das 4 Gew.-% Ozon enthält, mit der äquivalenten Menge an Ozon umgesetzt. Dabei wird Ozon quantitativ aufgenommen und der Restgehalt an p-Methylstyrol beträgt nach beendeter Ozonisierung weniger als 1 % der Ausgangskonzentration.

Die bei der Ozonisierung erhaltene Lösung wird über ein Dosiergefäß in einen Hydrierreaktor, in dem eine Suspension von 1 g Platin, hergestellt in situ durch Hydrierung von $PtO_2$, in 200 ml Methanol vorgelegt wird und der mit Wasserstoff gefüllt ist, in einer solchen Dosierung kontinuierlich eigespeist, daß der Peroxidgehalt in der Hydrierlösung zu Beginn und im Verlaufe der gesamten Hydrierung 0.02 Mol/l nicht übersteigt. Unter kräftigem Rühren und Wasserstoffzugabe wird bis zur negativen Peroxidprobe hydriert, wobei die Temperatur durch Kühlung von außen auf 30 bis 40°C gehalten wird. Der verbrauchte Wasserstoff wird laufend aus einem Vorratsgefäß ergänzt und durch Zugabe von methanolischen NaOH wird in der Lösung ein pH-Wert von 4 bis 5 eingehalten. Nachdem die Zugabe der Ozonisierungslösung beendet ist, kommt die Wasserstoffaufnahme in einigen Minuten zum Stillstand und die Reaktionslösung ist gänzlich peroxidfrei. Insgesamt werden während der Hydrierung 31.4 Normalliter Wasserstoff, entsprechend 93.4 % der Theorie aufgenommen.

Zur Aufarbeitung wird vom Katalysator abfiltriert und die Reaktionslösung durch Rühren mit einem stark sauren Ionenaustauscher (Lewatit) natriumfrei gemacht. Nach Abtrennen des Katalysators werden die Lösungsmittel und flüchtige Begleitprodukte am Rotovapor entfernt und der das Reaktionsprodukt enthaltende Rückstand im Vakuum rektifiziert.

Dabei werden 169 g p-Tolualdehyd vom $Kp_{10}$ = 106 - 108°C enthalten, entsprechend einer Ausbeute von 94 % der Theorie.

**Beispiel 2: p-Nitrobenzaldehyd**

224 g p-Nitrostyrol werden in 1 Liter Methanol gelöst und analog zu der in Beispiel 1 angegebenen Arbeitsweise mit Ozon umgesetzt und anschließend hydriert. Die Wasserstoffaufnahme beträgt 31.4 Normalliter entsprechend 93.4 % der Theorie.

Zur Aufarbeitung wird das Methanol zusammen mit flüchtigen Begleitprodukten am Rotovapor entfernt, der Rückstand wird in heißem Wasser gelöst und anschließend im Eisbad abgekühlt. Dabei kristallisieren 216 g reines p-Nitrobenzaldehyd vom Fp 105 - 106°C aus, entsprechend einer Ausbeute von 95.5 % der Theorie.

**Beispiel 3: Pyridin-4-aldehyd**

158 g 4-Vinylpyridin werden in 1 Liter Methanol gelöst und analog zu der in Beispiel 1 angegebenen Arbeitsweise mit Ozon umgesetzt und anschließend hydriert. Während der Hydrierung werden 29.8 Normalliter Wasserstoff aufgenommen, entsprechend 88.7 % der Theorie.

Die Aufarbeitung wird wie in Beispiel 1 durchgeführt und der das Reaktionsprodukt enthaltende Rückstand im Vakuum fraktioniert. Dabei werden 147 g Pyridin-4-aldehyd vom $Kp_{10}$ = 70 bis 72°C erhalten, entsprechend einer Ausbeute von 91,5 % der Theorie.

**Beispiel 4: Pyridin-2-aldehyd**

158 g 2-Vinylpyridin werden in 1 Liter Methanol gelöst und analog zu der in Beispiel 1 angegbenen Arbeitsweise mit Ozon umgesetzt und anschließend hydriert. Während der Hydrierung werden 28.9 Normalliter Wasserstoff aufgenommen, entsprechend 86 % der Theorie.

Die Aufarbeitung wird wie in Beispiel 1 durchgeführt und der das Reaktionsprodukt enthaltende Rückstand durch Rektifizieren gereinigt. Dabei werden 143 g Pyridin-2-aldehyd vom $Kp_{10}$ = 59 - 62°C erhalten, entsprechend einer Ausbeute von 89,1 % der Theorie.

**Beispiel 5: Brenztraubensäuremethylester**

150 g (1,5 mol) Methylmethacrylat werden in 1 l Methanol gelöst und bei -10 bis -5°C durch Einleiten eines Luft/$O_3$-Gemisches mit der äquivalenten Menge an Ozon umgesetzt. Nach Beendigung der Ozonisierung beträgt

der Restgehalt an Methylmethacrylat im Reaktionsgemisch weniger als 1 % der Ausgangskonzentration.

Im Hydrierreaktor wird eine Suspension von 1 g Platin in 200 ml Methanol vorgelegt und die Ozonisierungslösung während der Hydrierung unter starkem Rühren kontinuierlich in einer solchen Dosierung zugetropft, daß die Peroxidkonzentration in der Hydrierlösung eine Konzentration von 0.1 mol/l nicht übersteigt. Die Hydrierung erfolgt bei einer Temperatur von 30 bis 40°C und einem pH-Wert von 4 bis 5, der durch eine automatische Titration mit methanolischer Natronlauge eingestellt wird, und wird unter Ergänzung des verbrauchten Wasserstoffs bis zur negativen Peroxidprobe fortgesetzt. Der Wasserstoffverbrauch beträgt 32,2 Normalliter (96 % der Theorie).

Zur Aufarbeitung wird der Inhalt des Hydrierreaktors bis auf einen Rückstand von ca. 200 ml über eine Fritte abgesaugt. Der im Hydrierreaktor im kleineren Teil der Hydrierlösung zurückgebliebene Katalysator wird ohne Regenerierung oder Aufarbeitung für die erfindungsgemäße reduktive Spaltung weiter verwendet, indem von neuem ozonisierte Lösung von Methylmethacrylat über das Dosiergefäß in den Reaktor eingespeist und der Hydriervorgang unter den oben angegebenen Reaktionsbedingungen wiederholt wird. Insgesamt werden in fünf solcher Reaktionszyklen 7.5 Mol ozonisiertes Methylmethacrylat reduktiv aufgespalten. Der Gesamtwasserstoffverbrauch beträgt 159,2 Normalliter (94.8 % der Theorie). Zur Aufarbeitung werden die vereinigten Hydrierlösungen durch Rühren mit einem stark sauren Ionenaustauscher (Lewatit) natriumfrei gemacht und dann das Lösungsmittel zusammen mit dem während der Reaktion als Begleitprodukt in Form des Dimethylacetals anfallenden Formaldehyd im Vakuum entfernt. Der das Reaktionsprodukt enthaltende Rückstand wird durch Fraktionieren im Vakuum gereinigt. Dabei erhält man 698 g Brenztraubensäuremethylester vom $Kp_{40}$ = 61 - 62°C, entsprechend einer Ausbeute von 91.2 % der Theorie.

**Beispiel 6: Alpha-Ketobuttersäureäthylester**

192 g (1.5 mol) Äthylacrylsäureäthylester werden in 1 Liter Äthanol gelöst und analog zu der in Beispiel 5 angegebenen Arbeitsweise bei -35 bis -30°C mit Ozon umgesetzt und anschließend hydriert.

Der Wasserstoffverbrauch beträgt 32,4 Normalliter entsprechend 96.4 % der Theorie. Die Aufarbeitung wird wie in Beispiel 5 durchgeführt und bei der Vakuumrektifikation erhält man 171 g reinen alpha-Ketobuttersäureäthylester vom $Kp_{20}$ = 68 - 69°C, entsprechend einer Ausbeute von 87.7 % der Theorie.

**Beispiel 7: Mesoxalsäurediäthylester**

258 Diäthylmethylenmalonat, welches durch Knoevenagel-Kondensation von Malonester und Formaldehyd hergestellt wird, werden in 1 Liter Methanol gelöst und analog zu der in Beispiel 5 angegebenen Arbeitsweise mit Ozon umgesetzt und anschließend hydriert. Als Hydrierkatalysator werden 5 g 10 % Pd/C in 200 ml Methanol vorgelegt. Der Wasserstoffverbrauch beträgt 28.9 Normalliter, (86 % der Theorie). Nach der Aufarbeitung wie in Beispiel 5 und Rektifizieren im Vakuum werden 214 g Mesoxalsäurediäthylester vom $Kp_{20}$ = 110 - 112°C erhalten, entsprechend einer Ausbeute von 82 % der Theorie.

**Beispiel 8: 3,3-Dimethoxypropanal**

174 g (1.5 mol) 4,4-Dimethoxybuten werden in 1 Liter Methanol gelöst und unter Kühlung auf -10 bis 0°C durch Einleiten eines Ozon-Luftgemisches mit der äquivalenen Menge Ozon umgesetzt. Die Ozon-Aufnahme im Reaktionsgemisch ist quantitativ und der Gehalt an 4,4-Dimethoxybuten beträgt nach beendeter Ozonisierung weniger als 1 % der Ausgangsmenge.

In einem mit Wasserstoff gefüllten Hydrierreaktor wird eine Suspension von 0.5 g Pt in 200 ml Methanol vorgelegt und die Ozonisierungslösung in einer solchen Dosierrung zugetropft, daß der Peroxidgehalt in der Hydrierlösung zu Beginn und im Verlaufe der gesamten Hydrierung 0.05 Mol/l nicht übersteigt. Unter kräftigem Rühren und laufender Ergänzung des verbrauchten Wasserstoffs wird bei 35 - 40°C unter Einhaltung eines pH-Wertes von 3 bis 4, der durch automatische Titration mit methanolischer NaOH-Lösung kontrolliert wird, hydriert, wobei die Hydrierlösung nach der vollständigen Zugabe der Ozonisierungslösung in wenigen Minuten gänzlich peroxidfrei wird. Die Wasserstoffaufnahme beträgt 31.6 Normalliter, entsprechend 94 % der Theorie.

Der größte Teil der Hydrierlösung wird über eine Fritte aus dem Hydrierreaktor abgesaugt und das im Reaktor zusammen mit dem Katalysator zurückbleibende Restvolumen von etwa 200 ml wird neuerlich mit einer Peroxidlösung, welche durch Ozonisierung von 144 g 4,4-Dimethylbuten im Methanol hergestellt wurde, unter Einhaltung der oben angegebenen Peroxidkonzentration versetzt und hydriert.

Nach Beendigung der Hydrierung wird die gesamte Hydrierlösung aus dem Reaktor über eine Fritte abgesaugt, die Lösungen werden vereinigt und durch Behandeln mit einem stark sauren Ionenaustauscher natriumfrei gemacht. Das Methanol wird zusammen mit dem Hydrierwasser im Vakuum abgezogen und der Rückstand durch Rektifizieren gereinigt. Man erhält so 314 g reines 3,3-Dimethoxypropanal vom $Kp_{50}$ = 75°C, entsprechend einer Ausbeute von 89 % der Theorie.

**Beispiel 9: 3,3-Di-n-butoxypropanal**

300 g 4,4-Di-n-butoxybuten werden in 1 Liter Methanol gelöst und analog zu der in Beispiel 8 angegebenen Arbeitsweise mit Ozon umgesetzt und anschließend hydriert. Die Hydrierung wird so durchgeführt, das ein Gehalt an Peroxiden in der Hydrierlösung von 0.02 Mol/Liter nicht überschritten wird und ein pH-Wert zwischen 4 und 5 sowie eine Temperatur von 35°C eingehalten wird. Der Wasserstoffverbrauch beträgt 32,4 Normalliter, entsprechend 96 % der Theorie.

Nach dem Abtrennen des Katalysators wird wie in Beispiel 8 aufgearbeitet und man erhält 284 g 3,3-Di-n-

butoxypropanal vom $Kp_{50}$ = 86°C, entsprechend einer Ausbeute von 93,7 % der Theorie.

**Beispiel 10: Succindialdehyd**

81 g (0.75 mol) Cyclooctadien (1.5) werden mit Methanol auf ein Volumen von 1 Liter verdünnt. In diese Lösung wird bei -10 bis -5°C solange ein $O_2/O_3$-Gemisch, das 4 Gew.% Ozon enthält, eingeleitet, bis 1.5 Mol Ozon in die Lösung eingebracht worden sind. Zur Vermeidung von Verlusten flüchtiger Anteile wird das Abgas kondensiert und das Kondensat zur Ozonisierung zurückgeführt. Die Ozonaufnahme ist quantitativ.

In einem Hydrierreaktor werden 1 g Platin, hergestellt in situ durch Hydrierung von $PtO_2$ in 200 ml Methanol vorgelegt und die Suspension mit Wasserstoff überlagert. Die bei der Ozonisierung erhaltene Lösung wird unter starkem Rühren bei einer Temperatur von 30 bis 40°C kontinuierlich in solchen Dosen in die Hydrierlösung eingespeist, daß im Hydrierreaktor eine Peroxidkonzentration von 0.1 mol/l zu Beginn und im Verlaufe der Hydrierung nicht überschritten wird. Während der Hydrierung wird der verbrauchte Wasserstoff laufend ergänzt und der Verbrauch gemessen. Der pH-Wert in der Lösung wird durch automatische Titration mit methanolischer Natronlauge bei 2 - 5 gehalten. Wenige Minuten nachdem die Zugabe der Ozonisierungslösung beendet ist, kommt die Wasserstoffaufnahme zum Stillstand und die Lösung wird gänzlich peroxidfrei. Es werden 32,6 Normalliter Wasserstoff aufgenommen, entsprechend 97 % der Theorie.

Der Inhalt des Hydrierreaktors wird bis auf ein Restvolumen von etwa 200 ml, das auch den Katalysator enthält, abgesaugt und die oben beschriebene Ozonisierung und reduktive Spaltung wird noch zehnmal ohne Regenerierung oder Aufarbeitung des Katalysators wiederholt. Insgesamt werden so 8.25 mol Cyclooctadien (1.5) mit 16.5 mol Ozon umgesetzt. Der Gesamtverbrauch an Wasserstoff beträgt 355.3 Normalliter, entsprechend 96,1 % der Theorie. Die Gesamtausbeute an Succindialdehyd wird durch Oximtitration bestimmt und beträgt 15,8 mol (96 % der Theorie).

Die vom Katalysator befreiten vereinigten Reaktionslösungen werden durch Rühren mit einem stark sauren Ionenaustauscher natriumfrei gemacht und im Dünnschichtverdampfer im leichten Vakuum eingeengt. Zur Charakterisierung wird der Rückstand in Methanol unter saurer Katalyse acetalisiert und das dabei erhaltene Reaktionsgemisch im Vakuum fraktioniert. Man erhält so 2550 g 1,1,4,4-Tetramethoxybutan vom $Kp_{15}$ = 86 - 88°C, entsprechend einer Ausbeute vom 86,8 % der Theorie, neben Spurenmengen von 2,5-Dimethoxytetrahydrofuran.

**Beispiel 11: Adipinaldehyd**

123 g Cyclohexen werden in 1 Liter Methanol gelöst und analog zu der in Beispiel 10 angegebenen Arbeitsweise mit Ozon umgesetzt und anschließend hydriert. Das Abgas aus der Ozonisierung wird zur Rückgewinnung des flüchtigen Cyclohexens mit Methanol ausgewaschen und die methanolische Waschlösung zum Ozonisierungsreaktor zurückgeführt. Durch diese Maßnahme kann der Verlust an Cyclohexen so weit eingedämmt werden, daß 94 % der auf den Anfangsgehalt an Cyclohexen bezogenen theoretischen Peroxidmenge nach Ende der Ozonisierung in der Lösung vorhanden sind.

Der Wasserstoffverbrauch während der Hydrierung beträgt 30.5 Normalliter, entsprechend 96,7 % der Theorie bezogen auf die oben festgestellte Peroxidmenge.

Nach Abtrennen des Katalysators und Entfernen der Natriumionen durch Behandeln der Lösung mit einem stark sauren Ionenaustauscher wird durch Oximtitration eine Gesamtausbeute an Adipinaldehyd von 1.41 mol (94 % der Theorie) festgestellt. Zur Charakterisierung wird der Adipinaldehyd acetalisiert und man erhält 275 g 1,1,6,6-Tetramethoxyhexan vom $Kp_{20}$ = 111°C, entsprechend einer Ausbeute von 89 % der Theorie.

**Beispiel 12: 1,8-Octandial**

165 g (1.5 mol) Cycloocten werden wie in Beispiel 10 mit der äquivalenten Menge Ozon umgesetzt und anschließend hydriert. Die Wasserstoffaufnahme beträgt 32,2 Normalliter, entsprechend 95,8 % der Theorie. Nach Abtrennung des Katalysators ergibt die Oximtitration einen Gehalt an 1,8-Octandial von 1.42 mol (94 % der Theorie). Zur Charakterisierung und Isolierung des Reaktionsprodukts wird die methanolische Lösung des 1,8-Octandials durch Behandeln mit Mineralsäuren auf pH 1 eingestellt und zur Acetalbildung einige Stunden stehen gelassen.

Nachdem die Acetalbildung beendet ist, wird die Lösung in der Kälte neutralisiert und mit Wasser verdünnt. Dabei scheidet sich das 1,1,8,8-Tetramethoxyoctan als wasserunlösliches Öl ab und wird abgetrennt und die zurückbleibende Lösung mit Petroläther extrahiert. Die Produktphasen werden vereinigt, vom Petroläther befreit und im Vakuum fraktioniert. Man erhält so 322 g 1,1,8,8-Tetramethoxyoctan vom $Kp_{30}$ = 147 - 149°C, entsprechend 91,7 % der Theorie.

**Beispiel 13: 3-Thiaglutaraldehyd-3,3-dioxid**

177 g Sulfolen werden wie in Beispiel 10 mit Ozon umgesetzt und anschließend hydriert. Die $H_2$-Aufnahme beträgt 32,3 Normalliter, entsprechend 96,1 % der Theorie.

Nach Abtrennen des Katalysators ergibt die Oximtitration einen Gehalt von 2,87 mol Aldehydgruppen (95,8 % der Theorie).

Das Lösungsmittel wird am Rotovapor im Vakuum entfernt und man erhält 219 g eines hellgelben Rückstandes. Der Aldehydgruppengehalt pro Gramm entspricht 13.0 m mol, entsprechend einer Gesamtausbeute an 3-Thiaglutaraldehyd-3,3-dioxid von 94.9 % der Theorie.

**Beispiel 14: Homophthalaldehyd**

87 g Inden (0.75 mol) werden in 0.5 Liter Methanol gelöst und analog zu der in Beispiel 10 angegebenen Arbeitsweise ozonisiert und hydriert. Die $H_2$-Aufnahme beträgt 16,1 Normalliter. Nach Abtrennen des Katalysators ergibt die Aldehydgruppenbestimmung durch Oximtitration einen Gehalt von 41,1 mol (94 % der Theorie).

Ein Teil der Lösung wird am Rotovapor eingengt und der entstandene Homophtalaldehyd als Di-p-Nitrophenylhydrazon vom Fp 217 - 218°C charakterisiert.

**Beispiel 15: 1,6 Hexandial-3,4-dicarbonsäuredimethylester**

297 g (1.5 mol) Tetrahydrophthalsäuredimethylester werden in 1 Liter Methanol gelöst und wie in Beispiel 10 ozonisiert und hydriert. Die $H_2$-Aufnahme beträgt 31.4 Normalliter. Nach der Hydrierung und Abtrennen des Katalysators erhält man eine vollkommen peroxidfreie Lösung, deren Aldehydgruppenbestimmung durch Oximtitration einen Gehalt von 2.86 mol (95.3 % der Theorie) ergibt. Die Lösung wird durch Behandeln mit einem stark sauren Ionenaustauscher natriumfrei gemacht, die Produktlösung mit Wasser versetzt und Methanol abdestilliert. Dabei wird der Ester verseift und es werden 650 g einer wässerigen Lösung von 1,6 Hexandial-3,4-dicarbonsäure erhalten, die einen Aldehydgruppengehalt von 2,83 mol bzw. einen Carbonsäuregruppengehalt von 2,94 mol aufweist (94,3 % bzw. 98 % der Theorie, berechnet jeweils auf 1,6 Hexandial-3,4-dicarbonsäure).

**Beispiel 16: o-Phthaldialdehyd**

96 g (0,75 mol) Naphthalin werden in 1 Liter Methanol so weit als möglich gelöst und analog zu den Angaben in Beispiel 10 mit der äquivalenten Menge Ozon umgesetzt und anschließend hydriert. Während der Ozonisierung wird für eine gute Durchmischung gesorgt, damit für das abreagierte Naphthalin laufend noch nicht gelöstes Naphthalin in Lösung gebracht wird.

Nach Beendigung der Ozonisierung sind weniger als 2.5 % der ursprünglichen Naphthalinmenge in der Lösung vorhanden. Die Wasserstoffaufnahme während der Hydrierung beträgt 31,6 Normalliter, entsprechend 94 % der Theorie.

Zur Aufarbeitung wird vom Katalysator abfiltriert, das Methanol abdestilliert und der Rückstand in so viel heißem Wasser aufgenommen, das eine klare Lösung entsteht. Beim Stehen in der Kälte kristallisiert ein Teil der o-Phthaldialdehyds aus und wird abgetrennt. Die wässerige Phase wird zweimal mit Diäthyläther extrahiert, das vorher abgetrennte auskristallisierte Produkt in den vereinigten Ätherphasen gelöst und das organische Lösungsmittel abgedampft.

Es werden dabei 87 g (86.5 % der Theorie) o-Phthaldialdehyd als gelber Feststoff erhalten, dessen Aldehydgruppenbestimmung einen Gehalt von 14,8 m mol/g (99 % des theoretischen Aldehydgruppengehalts) ergibt und der einen unkorrigierten Schmelzpunkt von 54°C aufweist.

**Beispiel 17: 3-Oxaglutaraldehyd**

105 g 2,5-Dihydrofuran werden wie in Beispiel 10 bei -20°C mit Ozon umgesetzt und anschließend hydriert. Das Abgas aus der Ozonisierung wird zur Rückgewinnung des flüchtigen 2,5-Dihydrofurans mit Methanol ausgewaschen und die methanolische Waschlösung zum Ozonisierungsreaktor zurückgeführt. Durch diese Maßnahme kann der Verlust an 2,5-Dihydrofuran so weit eingedämmt werden, daß 95,3 % der auf den Anfangsgehalt an 2,5-Dihydrofuran bezogenen theoretischen Peroxidmenge nach Ende der Ozonisierung in der Lösung vorhanden sind.

Der Wasserstoffverbrauch während der Hydrierung beträgt 31,9 Normalliter, entsprechend 94,9 % der Theorie. In der Produktlösung ergibt die Oximtitration einen Aldehydgruppengehalt von 2,88 mol, entsprechend einer Ausbeute an 3-Oxaglutaraldehyd von 96 % der Theorie.

**Patentansprüche**

1. Verfahren zur Herstellung von Mono- oder Biscarbonylverbindungen der allgemeinen Formel

```
Q - X - C -R                                   I
        ||
        O
```

worin

```
                                               OR1
                                              /
Q  Wasserstoff oder die Reste - C - H,  - C - R1,  - CH      ,
                                ||       ||          \
                                O        O            OR1
- C - OR1
  ||
  O
```

bezeichnet, mit der Bedeutung für $R_1$ ist $C_1$ bis $C_6$ Alkyl,

X einen gegebenenfalls ein- oder mehrfach substituierten, geradkettigen oder verzweigten zweiwertigen aliphatischen Alkylenrest mit 1 bis 20 C-Atomen, wobei dieser Alkylenrest durch eine oder mehrere Gruppen, die unter den Reaktionsbedingungen inert sind, substituiert sein kann, einen geradkettigen oder verzweigten aliphatischen Alkylenrest mit 2 bis 20 C-Atomen, wobei eine der - $CH_2$ -Gruppen der Alkylenkette durch ein Sauerstoffatom, ein Schwefelatom oder eine - $SO_2$-Gruppe ersetzt ist,
einen gegebenenfalls ein- oder mehrfach substituierten Phenylenrest, wobei dieser Phenylenrest durch eine oder mehrere Gruppen, die unter den Reaktionsbedingungen inert sind, substituiert sein kann, einen gegebenenfalls ein- oder mehrfach substituierten zweiwertigen Aralkylen-, Alkylarylen- oder Alkylenarylenrest mit 7 bis 12 C-Atomen, wobei obgenannte Reste durch eine oder mehrere Gruppen, die unter den Reaktionsbedingungen inert sind, substituiert sein können, einen zweiwertigen Heterocyclus mit einem oder zwei Heteroatomen im Ring oder eine Einfachbindung zwischen zwei benachbarten C-Atomen bedeutet.

R Wasserstoff, einen $C_1$ bis $C_4$-Alkylrest oder den Rest 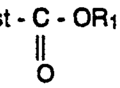

bezeichnet, wobei Glyoxal, Alkylglyoxale und deren Dialkylacetale aus der Formel I ausgenommen sind, durch Ozonisierung von ungesättigten organischen Kohlenstoffverbindungen mit einer oder mehreren olefinischen oder aromatischen Doppelbindungen im Molekül und anschließende katalytische Hydrierung der Ozonisierungsprodukte, dadurch gekennzeichnet ist, daß man

a) eine ungesättigte Verbindung der allgemeinen Formel

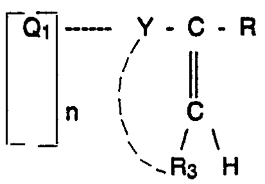

II

worin

n 0 oder 1 ist,
$Q_1$ Wasserstoff oder die Reste

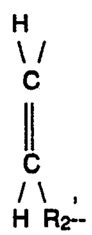 , 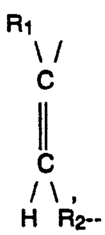 , 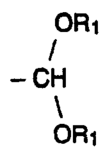 , 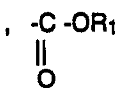

bezeichnet, mit der Bedeutung für $R_1$ ist $C_1$ bis $C_6$-Alkyl,

$R_2$ und $R_3$ unabhängig voneinander für Wasserstoff oder einen $C_1$ bis $C_4$-Alkylrest stehen oder, falls n 1 ist und $Q_1$ den Rest

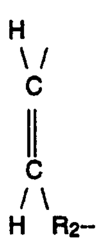

darstellt, stehen $R_2$ und $R_3$ zusammen für eine Einfachbindung zwischen zwei benachbarten C-Atomen oder für einen Alkylenrest mit 2 bis 4 C-Atomen, falls Y einen o-Phenylenrest oder enen Alkylenrest mit 2 bis 4 C-Atomen und R ein Wasserstoffatom bedeutet,

Y dieselbe Bedeutung wie X in Formel I hat, falls n 1 bedeutet, oder falls n für 0 steht, zusammen mit $R_3$ einen gegebenenfalls substituierten, zweiwertigen aliphatischen oder araliphatischen Rest, dessen aliphatische Kette durch

Sauerstoff oder Schwefel durchbrochen sein kann, bedeutet und R wie in Formell definiert ist, in einem niedrigen aliphatischen Alkohol bei Temperaturen von -80°C bis + 20°C mit der äquivalenten Menge Ozon umsetzt und

b) die dabei entstehende peroxidhältige Lösung in eine Suspension des Hydrierkatalysators in dem in Stufe a) bei der Ozonisierung verwendeten Alkohol kontinuierlich in einer solchen Dosierung einspeist, daß über den ganzen Verlauf der Hydrierung in der Hydrierlösung ein Peroxidgehalt von maximal 0.1 Mol/l eingestellt und/oder aufrechterhalten wird, und die Ozonisierungsprodukte bei pH 2 bis 7 und Temperaturen von 15 bis 45°C unter einem Druck von 1 bis 20 bar durch Hydrierung laufend in die entsprechende Carbonylverbindung reduktiv aufspaltet.

2. Verfahren nach Anspruch 1, dadurch gekennzeichnet, daß man die Ozonisierung in Stufe a) bei Temperaturen im Bereich von -15 bis 0°C durchführt.

3. Verfahren nach den Ansprüchen 1 und 2, dadurch gekennzeichnet, daß man bei der Ozonisierung in Stufe a) und der reduktiven Aufspaltung der Ozonisierungsprodukte in Stufe b) Methanol als Lösungsmittel verwendet.

4. Verfahren nach den Ansprüchen 1 bis 3, dadurch gekennzeichnet, daß zur reduktiven Aufspaltung der Ozonisierungsprodukte in Stufe b) in der Hydrierlösung ein Peroxidgehalt von maximal 0.02 Mol/l eingestellt und/oder aufrecht erhalten wird.

5. Verfahren nach den Ansprüchen 1 bis 4, dadurch gekennzeichnet, daß zur reduktiven Spaltung in Stufe b) Platin ohne Trägermaterial als Katalysator verwendet wird.

6. Verfahren nach den Ansprüchen 1 bis 5, dadurch gekennzeichnet, daß die reduktive Spaltung in Stufe b) im Temperaturbereich von 30 bis 40°C vorgenommen wird.

7. Verfahren nach den Ansprüchen 1 bis 6, dadurch gekennzeichnet, daß während der reduktiven Spaltung in Stufe b) ein pH-Wert von 3 bis 5 eingestellt wird.

8. Verfahren nach den Ansprüchen 1 bis 7, dadurch gekennzeichnet, daß man in Stufe b) einen Katalysator einsetzt, der bereits ein- oder mehrmals in einem Verfahren gemäß Anspruch 1 verwendet wurde.

9. Verfahren nach den Ansprüchen 1 bis 8, dadurch gekennzeichnet, daß ungesättigte Verbindungen der Formel

```
H - Y1 - C - H                IIa
         ||
         CH2
```

in der Y, zusammen mit Wasserstoff einen in ortho, meta- oder para-Stellung substituierten Phenylrest oder einen sechsgliedrigen Heterocyclus mit einem Heteroatom im Ring bedeutet, mit Ozon umgesetzt und anschließend reduktiv gespalten werden.

10. Verfahren nach Anspruch 9, dadurch gekennzeichnet, daß ungesättigte Verbindungen der Formel IIa, in der Y, zusammen mit Wasserstoff den p-Nitrophenyl, p-Tolyl-, 2 oder 4-Pyridinylrest bedeutet, mit Ozon umgesetzt und anschließend reduktiv gespalten werden.

11. Verfahren nach den Ansprüchen 1 bis 8, dadurch gekennzeichnet, daß ungesättigte Verbindungen der Formel

```
R4O - C - C - R5
      ||  ||                  IIb
      O   CH2
```

in der $R_4$ Methyl oder Äthyl und $R_5$ Methyl, Äthyl oder den Äthoxycarbonylrest bezeichnet, mit Ozon umgesetzt und anschließend reduktiv gespalten werden.

12. Verfahren nach Anspruch 11, dadurch gekennzeichnet, daß Methylmethacrylat mit Ozon umgesetzt und anschließend reduktiv gespalten wird.

13. Verfahren nach den Ansprüchen 1 bis 8, dadurch gekennzeichnet, daß ungesättigte Verbindungen der Formel

```
R1O
   \
    CH - CH2 - CH             IIc
   /           ||
R1O            CH2
```

in der $R_1$ wie in Formel I definiert ist mit Ozon umgesetzt und anschließend reduktiv gespalten werden.

14. Verfahren nach den Ansprüchen 1 bis 8, dadurch gekennzeichnet, daß Verbindungen der Formel in der $Y_2$ eine o-Phenylenrest oder Alkylenrest mit 2 bis 4 C-Atomen und $R_6$ und $R_7$ gemeinsam eine Einfachbindung

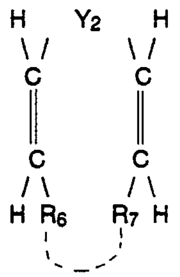

IId

zwischen zwei benachbarten C-Atomen oder einen Alkylenrest mit 2 bis 4-C-Atomen bedeuten, mit Ozon umgesetzt und anschließend reduktiv gespalten werden.

15. Verfahren nach den Ansprüchen 1 bis 8, dadurch gekennzeichnet, daß Verbindungen der Formel

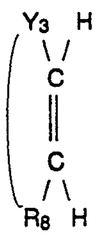

IIe

in der $Y_3$ und $R_8$ zusammen einen Alkylenrest mit 2 bis 6 C-Atomen oder die Reste $-CH_2-SO_2-CH_2-$, $-CH_2-O-CH_2-$,

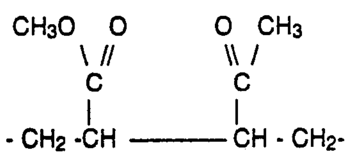 oder 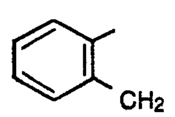 bedeuten, mit

Ozon umgesetzt und anschließend reduktiv gespalten werden.

## Claims

1. Process for the preparation of monocarbonyl or bis-carbonyl compounds of the general formula

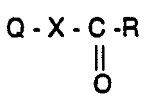

I

wherein Q denotes hydrogen or the radicals

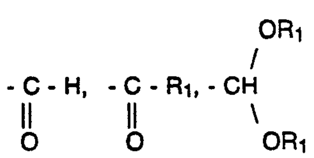

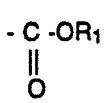

in which $R_1$ denotes $C_1$ to $C_6$ alkyl,

X denotes a linear or branched, divalent, aliphatic alkylene radical which has 1 to 20 C atoms and is optionally monosubstituted or polysubstituted, it being possible for this alkylene radical to be substituted by one or more groups which are inert under the reaction conditions, a linear or branched, aliphatic alkylene radical having 2 to 20 C atoms, one of the - $CH_2$ groups in the alkylene chain being replaced by an oxygen atom, a sulphur atom or an - $SO_2$- group, a phenylene radical which is optionally monosubstituted or polysubstituted, it being possible for this phenylene radical to be substituted by one or more groups which are inert under the reaction conditions, a divalent aralkylene, alkylarylene or alkylenearylene radical which has 7 to 12 C atoms and is optionally monosubstituted or polysubstituted, it being possible for the abovementioned radicals to be substituted by one or more groups which are inert under the reaction conditions, a divalent heterocyclic radical having 1 or 2 heteroatoms in the ring, or a single bond between two adjacent C atoms,

R denotes hydrogen, a $C_1$ to $C_4$ alkyl radical or the radical 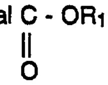, glyoxal, alkylglyoxals and dialkyl acetals being excepted from the formula I, by ozonizing unsaturated organic carbon compounds containing one or more olefinic or aromatic double bonds in the molecule and subsequently catalytically hydrogenating the ozonization products, characterized in that

a) an unsaturated compound of the general formula wherein

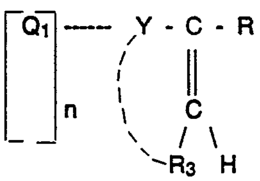

II

n is 0 or 1,
Q1 denotes hydrogen or the radicals

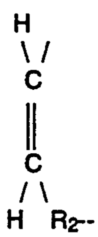 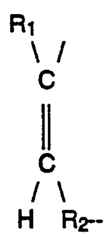 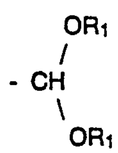 , 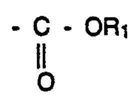

in which $R_1$ denotes $C_1$ to $C_8$ alkyl,
$R_2$ and $R_3$ independently of one another represent hydrogen or a $C_1$ to $C_4$ alkyl radical or, if n is 1 and $Q_1$ represents the radical

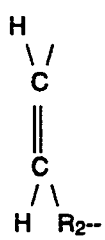

$R_2$ and $R_3$ together represent a single bond between two adjacent C atoms or an alkylene radical having 2 to 4 C atoms, if Y denotes an o-phenylene radical or an alkylene radical having 2 to 4 C atoms and R denotes a hydrogen atom,

Y has the same meaning as X in formula I if n denotes 1, or, if n represents 0, Y together with $R_3$ denotes an optionally substituted, divalent aliphatic or araliphatic radical in which the aliphatic chain can be interrupted by oxygen or sulfur, and R is as defined in formula I,
is reacted, in a lower aliphatic alcohol, at temperatures from -80°C to +20°C with the eqivalent amount of ozone,

b) the peroxide-containing solution thus formed is fed continuously into a suspension of hydrogenation catalyst in the alcohol used in the ozonization in stage a), at such a rate that, over the entire course of the hydrogenation, a peroxide content of not more than 0.1 mole/l is set up and/or maintained in the hydrogenation solution, and the ozonization products are continuously cleaved reductively into the corresponding carbonyl compound by hydrogenation at pH 2 to 7 and at temperatures from 15 to 45°C, under a pressure of 1 to 20 bar.

2. Process according to claim 1, characterized in that the ozonization in stage a) is carried out at temperatures within the range from -15 to 0°C.

3. Process according to claims 1 and 2, characterized in that methanol is used as the solvent in the ozonization in stage a) and in the reductive cleavage of the ozonization products in stage b).

4. Process according to claims 1 to 3, characterized in that a peroxide content of not more than 0.02 mole/l is set up and/or maintained in the hydrogenation solution for the reductive cleavage of the ozonization products in stage b).

5. Process according to claims 1 to 4, characterized in that platinum without a supporting material is used as the catalyst for the reductive cleavage in stage b).

6. Process according to claims 1 to 5, characterized in that the reductive cleavage in stage b) is carried out within the temperature range from 30 to 40°C.

7. Process according to claims 1 to 6, characterized in that the pH is adjusted to a value of 3 to 5 during the reductive cleavage in stage b).

8. Process according to claims 1 to 7, characterized in that in stage b) a catalyst is employed which has already been used once or several times in a process according to claim 1.

9. Process according to claims 1 to 8, characterized in that unsaturated compounds of the formula

```
H - Y1 - C - H
         ||                IIa
         CH2
```

in which $Y_1$ together with hydrogen denotes a phenyl radical which is substituted in the ortho-, meta- or paraposition or denotes a 6-membered heterocyclic structure having a hetero-atom in the ring, are reacted with ozone and are then cleaved reductively.

10. Process according to claim 9, characterized in that unsaturated compounds of the formula IIa in which $Y_1$ together with hydrogen denotes the p-nitrophenyl, p-tolyl, 2-pyridinyl or 4-pyridinyl radical are reacted with ozone and then cleaved reductively.

11. Process according to claims 1 to 8, characterized in that unsaturated compounds of the formula

```
                           IIb
R4O - C - C - R5
      ||  ||
      O   CH2
```

in which $R_4$ denotes methyl or ethyl and $R_5$ denotes methyl, ethyl or the ethoxycarbonyl radical are reacted with ozone and then cleaved reductively.

12. Process according to claim 11, characterized in that methyl methacrylate is reacted with ozone and then cleaved reductively.

13. Process according to claims 1 to 8, characterized in that unsaturated compounds of the formula

```
R1O
   \
   CH - CH2 - CH           IIc
   /          ||
R1O           CH2
```

in which $R_1$ is as defined in formula I are reacted with ozone and then cleaved reductively.

14. Process according to claims 1 to 8, characterized in that compounds of the formula

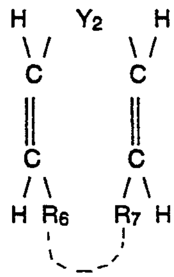

IId

in which $Y_2$ denotes an o-phenyl radical or an alkylene radical having 2 to 4 C atoms and $R_6$ and $R_7$ together denote a single bond between two adjacent C atoms or an alkylene radical having 2 to 4 C atoms are reacted with ozone and then cleaved reductively.

15. Process according to claims 1 to 8, characterized in that compounds of the formula

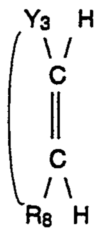

IIe

in which $Y_3$ and $R_8$ together denote an alkylene radical having 2 to 6 C atoms or the radicals $-CH_2-SO_2-CH_2-$, $-CH_2-O-CH_2-$,

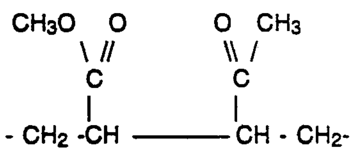 or 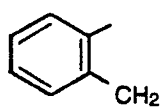

are reacted with ozone and then cleaved reductively.

**Revendications**

1. Procédé pour la préparation de composés mono- ou biscarbonyliques de formule générale

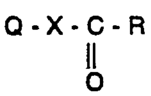

I

dans laquelle

Q désigne un atome d'hydrogène ou les radicaux

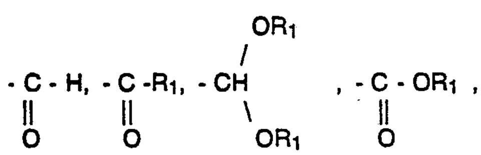

dans lesquels R a la signification d'un groupe $C_1$ à $C_6$-alcoyle,

X signifie un radical alcoylène aliphatique divalent linéaire ou ramifié éventuellement mono- ou polysubstitué ayant 1 à 20 atomes de carbone, ce radical alcoylène pouvant être substitué par un ou plusieurs groupes qui sont inertes

dans les conditions de réaction, un radical alcoylène aliphatique linéaire ou ramifié ayant 2 à 20 atomes de carbone, l'un des groupes $-CH_2-$ de la chaîne alcoylène étant remplacé par un atome d'oxygène, un atome de soufre ou un groupe $-SO_2-$, un radical phényle éventuellement mono- ou polysubstitué, ce radical phényle pouvant être substitué par un ou plusieurs groupes qui sont inertes dans les conditions de réaction, un radical arylalcoylène, alcoylarylène ou alcoylènearylène divalent éventuellement mono- ou polysubstitué ayant 7 à 12 atomes de carbone, les radicaux précités pouvant être substitués par un ou plusieurs groupes qui sont inertes dans les conditions de réaction, un hétérocycle divalent ayant un ou deux hétéroatomes dans le noyau ou une liaison simple entre deux atomes de carbone contigus,

R désigne un atome d'hydrogène, un radical $C_1$ à $C_4$ -alcoyle ou le radical 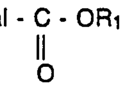,

le glyoxal, les alcoylglyoxals et leurs dialcoylacétals étant exceptés de la formule I,

par ozonisation de composés hydrocarbonés organiques insaturés ayant une ou plusieurs doubles liaisons oléfiniques ou aromatiques dans la molécule et hydrogénation catalytique consécutive des produits, caractérisé en ce que

a) on fait réagir avec la quantité équivalente d'ozone dans un alcool aliphatique inférieur aux températures de -80°C à +20°C, un composé insaturé de formule générale

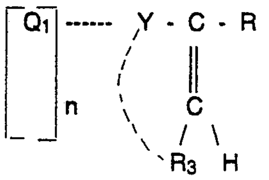

II

dans laquelle

n est 0 ou 1,

$Q_1$ désigne un atome d'hydrogène ou les radicaux

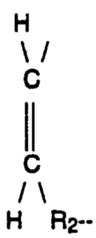 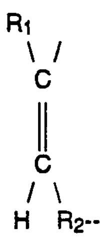 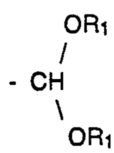 , 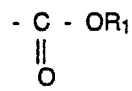

dans lesquels $R_1$ a la signification d'un groupe $C_1$ à $C_6$ - alcoyle, $R_2$ et $R_3$ dénotent indépendamment l'un de l'autre un atome d'hydrogène ou un radical $C_1$ à $C_4$ -alcoyle ou, au cas où n est 1 et Q représente

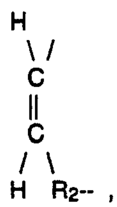

le radical $R_2$ et $R_3$ dénotent conjointement une liaison simple entre deux atomes de carbone contigus ou dénotent un radical alcoylène ayant 2 à 4 atomes de carbone, au cas où Y signifie un radical o-phénylène ou un radical alcoylène ayant 2 à 4 atomes de carbone et R signifie un atome d'hydrogène,

Y a la même signification que X dans la formule I, au cas où n signifie 1, ou au cas où n dénote 0, signifie conjointement avec $R_3$ un radical aliphatique ou arylaliphatique divalent éventuellement substitué, dont la chaîne aliphatique peut être interrompue par un atome d'oxygène ou de soufre, et R est défini comme dans la formule I, et

b) on alimente en continu avec la solution contenant un peroxyde formée dans ce cas, une suspension du catalyseur d'hydrogénation dans l'alcool utilisé dans le stade a) lors de l'ozonisation, en un dosage tel que pendant toute la durée de l'hydrogénation de la solution d'hydrogénation, une teneur en peroxyde d'au maximum 0,1 mole/l soit réglée et/ou maintenue, et on effectue de manière continue le clivage réducteur par hydrogénation des produits d'ozonisation, à pH 2 à 7 et aux températures de 15 à 45°C sous une pression de 1 à 20 bars, en composé carbonylique correspondant.

2. Procédé selon la revendication 1, caractérisé en ce qu'on effectue l'ozonisation dans le stade a) aux températures dans l'intervalle de - 15 à 0°C.

3. Procédé selon les revendications 1 et 2, caractérisé en ce que, lors de l'ozonisation dans le stade a) et du clivage réducteur des produits d'ozonisation dans le stade b), on utilise le méthanol comme solvant.

4. Procédé selon les revendications 1 à 3, caractérisé en ce que, pour le clivage réducteur des produits d'ozonisation dans le stade b), on règle et/ou on maintient dans la solution d'hydrogénation une teneur en peroxyde d'au maximum 0,02 mole/l.

5. Procédé selon les revendications 1 à 4, caractérisé en ce que, pour le clivage réducteur dans le stade b), on utilise le platine sans matériau de support comme catalyseur.

6. Procédé selon les revendications 1 à 7, caractérisé en ce que l'on effectue le clivage réducteur dans le stade b) dans l'intervalle de températures de 30 à 40°C.

7. Procédé selon les revendications 1 à 6, caractérisé en ce que pendant le clivage réducteur dans le stade b), on règle une valeur de pH de 3 à 5.

8. Procédé selon les revendications 1 à 7, caractérisé en ce que dans le stade b), on introduit un catalyseur qui a déjà été utilisé une ou plusieurs fois dans un procédé selon la revendication 1.

9. Procédé selon les revendications 1 à 8, caractérisé en ce qu'on fait réagir avec l'ozone les composés insaturés de formule

```
H - Y1 - C - H                IIa
         ||
         CH2
```

dans laquelle $Y_1$ signifie conjointement avec un atome d'hydrogène, un radical phényle substitué en position ortho, méta ou para ou un hétérocycle ayant six chaînons dans le noyau, et on les clive consécutivement par voie de réduction.

10. Procédé selon la revendication 9, caractérisé en ce qu'on fait réagir avec l'ozone les composés insaturés de formule IIa, dans laquelle $Y_1$ signifie conjointement avec un atome d'hydrogène le radical p-nitrophényle, p-tolyle, 2- ou 4-pyridinyle, et on les clive consécutivement par voie de réduction.

11. Procédé selon les revendications 1 à 8, caractérisé en ce qu'on fait réagir avec l'ozone les composés insaturés de formule

```
R4O - C - C -R5               IIb
      ||  ||
      O   CH2
```

dans laquelle $R_4$ désigne un radical méthyle ou éthyle et $R_5$ désigne un radical méthyle, éthyle ou éthoxycarbonyle, et on les clive consécutivement par voie de réduction.

12. Procédé selon la revendication 11, caractérisé en ce qu'on fait réagir avec l'ozone le méthacrylate de méthyle, et on le clive consécutivement par voie de réduction.

13. Procédé selon les revendications 1 à 8, caractérisé en ce qu'on fait réagir avec l'ozone les composés insaturés de formule

```
R1O
   \
    CH - CH2 - CH             IIc
   /           ||
R1O            CH2
```

dans laquelle $R_1$ est défini comme dans la formule I, et on les clive consécutivement par voie de réduction.

14. Procédé selon les revendications 1 à 8, caractérisé en ce qu'on fait réagir avec l'ozone les composés de formule

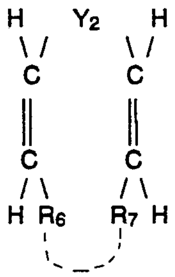

IId

dans laquelle $Y_2$ signifie un radical o-phénylène ou un radical alcoylène ayant 2 à 4 atomes de carbone et $R_6$ et $R_7$ signifient en commun une liaison simple entre deux atomes de carbone contigus ou un radical alcoylène ayant 2 à 4 atomes de carbone, et on les clive consécutivement par voie de réduction.

15. Procédé selon les revendications 1 à 8, caractérisé en ce qu'on fait réagir avec l'ozone les composés de formule

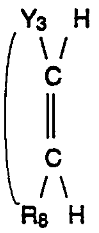

IIe

dans laquelle $Y_3$ et $R_8$ signifient conjointement un radical alcoylène ayant 2 à 6 atomes de carbone ou les radicaux

$-CH_2-SO_2-CH_2-$, $-CH_2-O-CH_2-$,

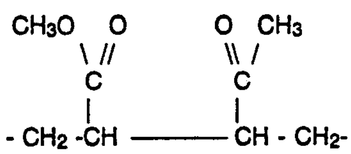 ou 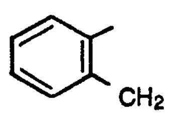

et on les clive consécutivement par voie de réduction.